# EUROPEAN PATENT SPECIFICATION

(11) **EP 3 617 695 B1**
(45) Date of publication and mention of the grant of the patent: **02.10.2024**
(21) Application number: 18781945.3
(22) Date of filing: 19.04.2018
(51) Int. Cl.: A01K 43/04, G01N 21/85, G01N 21/95

(54) **EGG INSPECTION DEVICE**
EI-INSPEKTIONSVORRICHTUNG
DISPOSITIF D'INSPECTION D' UFS

(30) Priority: 28.04.2017 JP 2017090273
(43) Date of publication of application: 04.03.2020
(73) Proprietor: Nabel Co., Ltd., Kyoto-shi Kyoto 601-8444 (JP)
(72) Inventor: HASEGAWA, Takayuki, Kyoto-shi Kyoto 601-8444 (JP)
(74) Representative: Prüfer & Partner mbB Patentanwälte · Rechtsanwälte
(86) International application number: PCT/JP2018/016105
(87) International publication number: WO 2018/198923

(56) References cited:
- WO-A1-2017/029864
- WO-A1-2017/029864
- JP-A- 2001 327 226
- JP-A- 2001 509 895
- JP-A- 2009 537 422
- JP-A- 2014 000 047
- JP-A- 2014 000 047
- US-A- 5 745 228
- US-A- 5 845 002
- US-A1- 2010 026 989
- US-A1- 2010 141 933
- US-A1- 2010 141 933

## Description

### TECHNICAL FIELD

The present invention relates to an egg inspection apparatus, and more particularly to an egg inspection apparatus to determine a condition of a surface of an egg.

### BACKGROUND ART

US 5 845 002 A discloses a method and an apparatus for detecting surface features of translucent objects by using two cameras, upstream and downstream from each other, each having two slanting illumination units illuminating the translucent objects from below.

WO 2017/029864 A1 discloses an egg inspection device with an ultraviolet light irradiation means, an ultraviolet light imaging means, a visible light irradiation means or an infrared light irradiation means and a transmission imaging means. Cameras are imaging the eggs from both ends.

JP 2001 327226 A discloses an egg inspection and an egg treatment apparatus using two cameras being positioned upstream and downstream relative to each other. Two corresponding illumination devices illuminate the sharp ends of the eggs.

US 2010/0026989 A1 discloses an egg inspection apparatus comprising two cameras imaging both ends of the eggs during a transport along a transport path while the eggs are rotated. A plurality of slanted laser rows are positioned downstream the transport path below a conveyor used for said transport, whereby either a central region or one of the ends of the egg are illuminated. The rows of lasers are switched on and off in synchronisation with the transport position of the eggs.

Eggs to be shipped to the market are graded by a grading and packing system, depending on physical properties such as weight, and thereafter packed in a transparent package made of synthetic resin or the like. Prior to or in parallel with thus being graded and packed, the eggs undergo various inspections. One such inspection is performed to screen eggs having surfaces in some conditions (or eggshells having some physical properties), more specifically, eggs having surfaces broken/cracked or with chicken feces adhering thereto.

For example, PTD 1 discloses an egg inspection apparatus comprising an illumination device, an imaging device, and a determination unit. The illumination device is disposed on a roller in a frontward and rearward direction and a rightward and leftward direction, and emits light directly from below toward eggs transported while being rotated about their respective longer axes. The imaging device images an egg directly from above. The determination unit determines a condition of a surface of each egg based on the obtained image.

### CITATION LIST

### PATENT DOCUMENT

PTD1: Japanese Patent Laying-Open No. 11-326202

### SUMMARY OF INVENTION

### TECHNICAL PROBLEM

While such an egg inspection apparatus as described above can inspect a condition of a surface around an equatorial region of an egg, the egg inspection apparatus may overlook breakage, a crack, dirt and the like present on the side of the small end or large end of the egg. When observing an egg up to an end thereof, an imaging device used to image the egg may be oriented obliquely. However, simply with such an arrangement, it is expected that the light emitted from the illumination device leaks from an outside of the egg and enters the imaging device, and a portion of an image which appears white is erroneously determined as a crack.

The present invention has been made to solve the above described, expected problem and it is an object of the present invention to provide an egg inspection apparatus capable of inspecting whether an egg has a small or large end having a surface in a poor condition, such as a broken, cracked, dirty or similar surface.

### SOLUTION TO PROBLEM

The above mentioned object is achieved by the egg inspection apparatus according to claim 1.

### ADVANTAGEOUS EFFECTS OF INVENTION

The egg inspection apparatus according to the present invention can thus inspect whether an egg has a small end or a large end having a broken, cracked, dirty or similarly defective surface.

### BRIEF DESCRIPTION OF DRAWINGS

Fig. 1 is a block diagram showing an example of a configuration of an egg grading system to which an egg inspection apparatus according to an embodiment is applied.
Fig. 2 is a partial plan view showing the egg inspection apparatus according to the embodiment of the present invention.
Fig. 3 is a partial side view of the egg inspection apparatus shown in Fig. 2, as seen in the conveying direction, for illustrating an operation of the egg inspection apparatus in the embodiment.
Fig. 4 is a partial bottom view of a conveyor unit of the egg inspection apparatus shown in Fig. 2 in the same embodiment, as viewed from below.
Fig. 5 is a diagram schematically showing an example of an image of an egg obtained by the egg inspection apparatus in the same embodiment.
Fig. 6 is a first partial side view for illustrating an operation of the egg inspection apparatus in the same embodiment.
Fig. 7 is a second partial side view for illustrating an operation of the egg inspection apparatus in the same embodiment.

### DESCRIPTION OF EMBODIMENTS

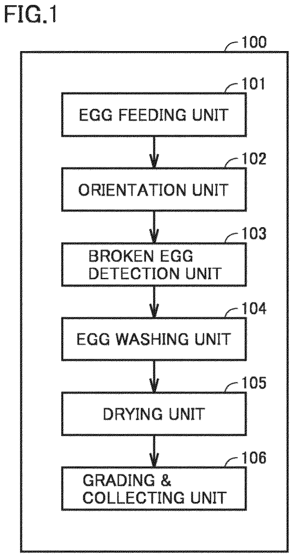

Hereinafter, an egg inspection apparatus according to an embodiment of the present invention will be described with reference to Figs. 1 to 7. An egg inspection apparatus 1 according to the present embodiment is used in an egg grading system for grading eggs depending on physical properties such as egg weight.

As shown in Fig. 1, an egg grading system 100 comprises, for example, an egg feeding unit 101, an orientation unit 102, a broken egg detection unit 103, an egg washing unit 104, a drying unit 105, and a grading and collecting unit 106. Eggs are fed to egg feeding unit 101. Orientation unit 102 aligns a plurality of fed eggs E in direction. Broken egg detection unit 103 mainly detects among the aligned eggs E an egg which has a broken eggshell (hereinafter also referred to as a "broken egg"). Egg inspection apparatus 1 according to one embodiment corresponds to broken egg detection unit 103. Egg washing unit 104 washes eggs E other than broken eggs with warm water or the like. Drying unit 105 dries a surface of egg E washed. Grading and collecting unit 106 employs a known spectroscopic analysis processing technique or acoustic processing technique to detect physical properties of the washed and dried eggs E, select a cracked egg, a dirt egg, a blood egg, a spoiled egg, an extremely small egg, an extremely large egg, and other eggs departing from chicken egg trading standards, and exclude them from those to be graded. Grading and collecting unit 106 measures eggs in weight, and, based on a result thereof, grades the eggs for each weight category of agricultural standards. For egg feeding unit 101, orientation unit 102, egg washing unit 104, drying unit 105 and grading and collecting unit 106, except for broken egg detection unit 103 (or egg inspection apparatus 1), a variety of known units are applicable.

Egg inspection apparatus 1 corresponding to broken egg detection unit 103 is disposed upstream of the washing unit for washing egg E and the drying unit for drying egg E. Hereinafter, egg inspection apparatus 1 according to the present embodiment will be described in detail.

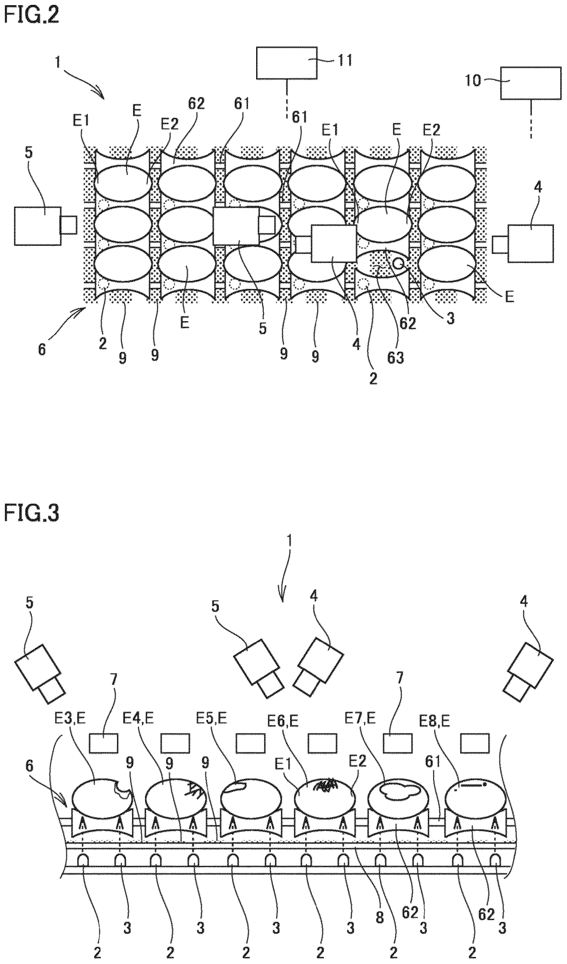

As shown in Figs. 2 and 3, egg inspection apparatus 1 includes a first irradiation unit 2 and a second irradiation unit 3, a first imaging unit 4 and a second imaging unit 5, and a determination unit 10. First irradiation unit 2 and second irradiation unit 3 irradiate egg E with light. First imaging unit 4 and second imaging unit 5 image egg E irradiated with light. Determination unit 10 determines a condition of a surface of egg E based on the obtained image of egg E. Egg inspection apparatus 1 inspects egg E transported downstream while being rotated by conveyor unit 6. A series of operations and the like of egg inspection apparatus 1 are controlled by a control unit 11.

As shown in Figs. 2 to 4, 6 and 7, first irradiation unit 2 emits light toward egg E from the one end E1 side of egg E. Second irradiation unit 3 emits light toward egg E from the other end E2 side of egg E. First irradiation unit 2 and second irradiation unit 3 are disposed below conveyor unit 6 and emits infrared light toward the side of the lower surface of egg E. In the present embodiment, as first irradiation unit 2 and second irradiation unit 3, for example, an LED (light emitting diode) emitting infrared light having a wavelength of 750 nm to 850 nm is used.

First irradiation unit 2 and second irradiation unit 3 emit light based on a signal issued from a sensor 7 provided upstream of first irradiation unit 2 and second irradiation unit 3 to sense whether egg E is present or absent. Sensor 7 is disposed above egg E and senses egg E transported from upstream by conveyor unit 6. When egg E sensed by sensor 7 is transported to a predetermined position, first irradiation unit 2 and second irradiation unit 3 emit light. Therefore, first irradiation unit 2 and second irradiation unit 3 emit light intermittently.

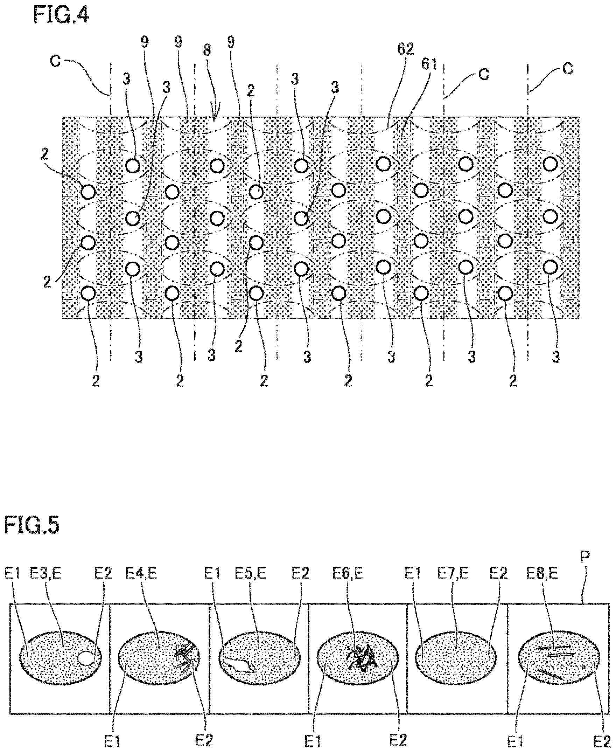

As shown in Figs. 2 and 4, egg inspection apparatus 1 according to the present embodiment has three first irradiation units 2 aligned in the conveying direction with a predetermined spacing therebetween. Three second irradiation units 3 are disposed at a location offset from first irradiation units 2 by half a pitch such that second irradiation units 3 are aligned in the conveying direction with a predetermined spacing therebetween. First irradiation unit 2 is disposed at a position offset to one side from a center C in the rightward and leftward direction of each of six rows of conveyor unit 6. Second irradiation unit 3 is disposed at a position offset to the other side from center C in the rightward and leftward direction of each of the six rows of conveyor unit 6. That is, first irradiation unit 2 and second irradiation unit 3 are staggered in the conveying direction of egg E.

Each first irradiation unit 2 is set at a position obstructed by egg E on conveyor unit 6, as seen in an image P obtained by first imaging unit 4, as will be described later. Each second irradiation unit 3 is set at a position obstructed by egg E on conveyor unit 6, as seen in image P obtained by second imaging unit 5, as will be described later. In egg inspection apparatus 1, the imaging unit includes first imaging unit 4 and second imaging unit 5, and the irradiation unit includes first irradiation unit 2 and second irradiation unit 3. First imaging unit 4 images egg E from the other end E2 side of egg E. Second imaging unit 5 images egg E from the one end E1 side of egg E. First irradiation unit 2 is disposed on an extension line of a line connecting first imaging unit 4 and egg E placed on conveyor unit 6 (or an opening 63 of conveyor unit 6 described later). Second irradiation unit 3 is disposed on an extension line of a line connecting second imaging unit 5 and egg E placed on conveyor unit 6 (or opening 63 of conveyor unit 6 described later).

As shown in Figs. 2, 3, 6, and 7, egg inspection apparatus 1 includes first imaging unit 4 and second imaging unit 5. First imaging unit 4 images egg E from the other end E2 side of egg E. Second imaging unit 5 images egg E from the one end E1 side of egg E. First imaging unit 4 and second imaging unit 5 are disposed above conveyor unit 6, first irradiation unit 2, and second irradiation unit 3 obliquely. First imaging unit 4 and second imaging unit 5 can image an upper surface of egg E. In egg inspection apparatus 1 according to the present embodiment, as first imaging unit 4 and second imaging unit 5, a CCD (Charge Coupled Device) camera having sensitivity for the near infrared range is used. Thus, image P of egg E irradiated with infrared light, including infrared light transmitted through egg E, can be obtained. First imaging unit 4 and second imaging unit 5 are set to include three rows of eggs E in a field of view. In egg inspection apparatus 1, for example, two first imaging units 4 and two second imaging units 5 are disposed. The units are not limited in number as above.

Fig. 5 schematically shows an example of image P of egg E shown in Fig. 3 obtained by first imaging unit 4 and second imaging unit 5. In egg inspection apparatus 1 according to the present embodiment, image P corresponding to eggs of 3 rows and 3 columns is obtained. Herein, for the sake of illustration, an image of six eggs picked up from image P is shown. Fig. 5 schematically shows, successively from left to right, an image of an egg with a hole E3, a recessed egg E4, an open cracked egg E5, an egg with feces E6 adhering thereto, an egg with egg contents adhering thereto E7, and an internally damaged/spotted egg E8. Eggs having eggshells with a large hole, such as egg with a hole E3 and open cracked egg E5, are subject to disposal. In contrast, recessed egg E4 can be set not to be discarded unless it has a hole. Egg with feces E6 adhering thereto, egg with egg contents (i.e., egg yolk, egg white, and the like having leaked out of another egg) adhering thereto E7, and internally damaged/spotted egg E8 are set not to be discarded.

Determination unit 10 (see Fig. 2) determines a condition of a surface of egg E based on image P obtained. It should be noted that determination unit 10 is not specifically limited in manner to the manner described hereinafter and a variety of image processing techniques may be applied.

Egg inspection apparatus 1 is controlled by control unit 11, which is a microcomputer system having a processor, a memory, an input interface, an output interface, and the like. Control unit 11 has a memory, which stores a predetermined threshold value for comparing obtained image P with a gray value converted into a numerical value. As the predetermined threshold value, for example, a threshold value for determining "a crack" or "adhesion of feces" or the like for image P of an egg irradiated with infrared light would be considered.

If image P obtained has a gray value falling within a range smaller (or darker) than the predetermined threshold value, it can be determined that the egg has no "crack." On the other hand, if image P obtained has a portion having a gray value larger (or brighter) than the predetermined threshold value, it can be determined that the egg has the portion "cracked." That is, it can be said that a bright region in image P is a portion which transmits more infrared light.

Infrared light with which an egg is irradiated penetrates the eggshell and is scattered inside the eggshell. When the infrared light scattered inside the eggshell again passes through the eggshell, the entire egg E acts like a luminous body and thus emits infrared light. In doing so, egg E at a cracked portion transmits more infrared light than at uncracked portion by the crack of the eggshell. Accordingly, the cracked portion will be displayed brighter in the image P than the uncracked portion.

As a specific method for subjecting each image P to image processing, each image P may be binarized using a predetermined threshold value. If there is a portion with a "crack" in image P, that portion appears as a brighter (or white) area than other portions. Determination unit 10 may determine that there is a "crack" if there is any bright area, or determination unit 10 may do so when an area ratio of a bright area to a reference area exceeds a threshold value. A portion with feces adhering thereto is also displayed as a darker (or black) area than other portions by performing a similar process using a different threshold value.

As shown in Figs. 2 and 3, in egg inspection apparatus 1, conveyor unit 6 transports a plurality of eggs E in alignment. Conveyor unit 6 includes a roller 62 and a roller shaft 61. Roller 62 is in the form of a spool. A plurality of rollers 62 are attached on roller shaft 61 in a direction orthogonal to the conveying direction. Egg E will be held between rollers 62s adjacent to each other in the conveying direction. At this location, a vertically penetrating opening 63 (see Fig. 2) is formed. Egg E rotates in a direction opposite to that in which roller 62 rotates. In Fig. 4, roller shaft 61 and roller 62 are indicated by a two-dot chain line.

As shown in Fig. 3 and Fig. 4, below conveyor unit 6, a protective member 8 is provided to prevent dust, contents of egg E and/or the like from falling directly on first irradiation unit 2 and second irradiation unit 3. Protective member 8 is in the form of a plate and is disposed so as to cover entirely a region in which first irradiation unit 2 and second irradiation unit 3 are disposed. Protective member 8 is formed of a material which transmits infrared light. In Fig. 3, paths of light emitted from first irradiation unit 2 and second irradiation unit 3 are schematically shown by dotted arrows.

Further, as shown in Figs. 2 to 4, a black reflection suppressing portion 9 is provided below conveyor unit 6. Reflection suppressing portion 9 is provided on the side of the upper surface of protective member 8. It can weaken light emitted from first irradiation unit 2 and second irradiation unit 3 and having reached conveyor unit 6, and reflected by egg E or a lower side of conveyor unit 6, and thus suppress re-arrival of the light at conveyor unit 6. In Figs. 2 to 4, reflection suppressing portion 9 is indicated by dots. Reflection suppressing portion 9 is disposed so as not to interrupt the paths of light emitted from first irradiation unit 2 and second irradiation unit 3 toward eggs E. Reflection suppressing portion 9 is disposed in the form of a plurality of strips in the conveying direction.

Hereinafter will be described one example of how in egg inspection apparatus 1 according to the present embodiment first irradiation unit 2 and second irradiation unit 3, and first imaging unit 4 and second imaging unit 5 are controlled.

Initially, whether an egg E to be inspected is present or absent is sensed. Specifically, a sensor disposed upstream of first irradiation unit 2 and second irradiation unit 3 by a predetermined distance is used to detect whether egg E is disposed between rollers 62s adjacent in the conveying direction. A detection result signal is output.

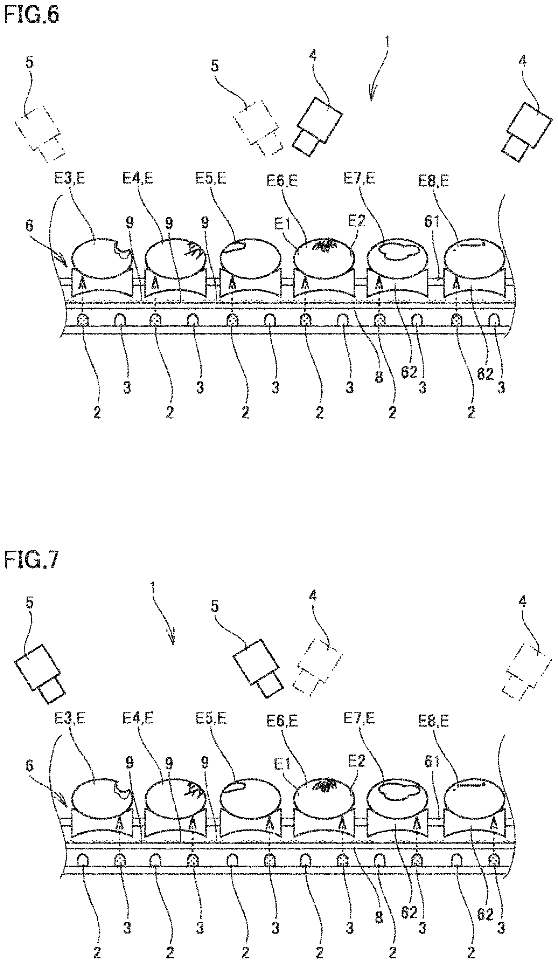

When egg E to be inspected is detected, and transported to a position exactly above first irradiation unit 2, then, as shown in Fig. 6, first irradiation unit 2 is turned on to emit infrared light toward egg E. If the emitted infrared light directly enters first imaging unit 4 or second imaging unit 5, it will cause a problem in imaging egg E. Accordingly, it is desirable to set where or when to emit infrared light by first irradiation unit 2 to allow the infrared light to irradiate the eggshell within an appropriate area. In accordance with the emission of infrared light, first imaging unit 4 images egg E irradiated with infrared light. At the time, second irradiation unit 3 is turned off.

Subsequently, conveyor unit 6 transports egg E to a position exactly above second irradiation unit 3, when second irradiation unit 3 is turned on to emit infrared light toward egg E, as shown in Fig. 7. In accordance with the emission of infrared light, second imaging unit 5 images egg E irradiated with infrared light. At the time, first irradiation unit 2 is turned off.

That is, in egg inspection apparatus 1, first imaging unit 4 images egg E on the other end E2 side thereof when first irradiation unit 2 emits infrared light toward egg E and second irradiation unit 3 does not emit infrared light toward egg E. In contrast, second imaging unit 5 images egg E on the one end E1 side thereof when first irradiation unit 2 does not emit infrared light toward egg E and second irradiation unit 3 emits infrared light toward egg E.

It should be noted that in these steps, first imaging unit 4 images a single rotating and moving egg E three times, and so does second imaging unit 5. Thus, the egg will be imaged repeatedly six times in total. This can inspect the condition of the entire circumferential surface of egg E.

As has been described above, egg inspection apparatus 1 according to the present embodiment includes a plurality of irradiation units, a plurality of imaging units, and a determination unit 10. The plurality of irradiation units include first irradiation unit 2 and second irradiation unit 3. The plurality of imaging units include first imaging unit 4 and second imaging unit 5. First irradiation unit 2 emits light toward egg E from the one end E1 side of egg E. Second irradiation unit 3 emits light toward egg E from the other end E2 side of egg E. First imaging unit 4 images egg E from the other end E2 side of egg E when first irradiation unit 2 emits infrared light toward egg E. Second imaging unit 5 images egg E from the one end E1 side of egg E when second irradiation unit 3 emits infrared light toward egg E.

Whether egg E has a small end or a large end having a broken, cracked, dirty or similarly defective surface, can thus be inspected. In particular, egg E is transported while it is rotated about its longer axis by roller 62, and egg E is thus circumferentially inspected successively. First imaging unit 4 images egg E in an area from the other end E2 of egg E to an equatorial region of egg E. Second imaging unit 5 images egg E in an area from the one end E1 of egg E to the equatorial region of egg E. Thus, egg E can also be inspected for whether it has a crack or the like in the vicinity of the equatorial region. This can automate conventional human visual inspection of eggs broken or having their eggshells cracked and their contents leaking out, and thus contribute to reduction of personnel expenses.

In particular, egg inspection apparatus 1 described above has first irradiation unit 2 disposed on the end E1 side of egg E, and first imaging unit 4 disposed on a side opposite to end E1 of egg E, or the end E2 side. Egg E will be located on a line connecting first imaging unit 4 and first irradiation unit 2. With this arrangement, when egg E irradiated with light by first irradiation unit 2 is imaged by first imaging unit 4, egg E will obstruct a component of light emitted from first irradiation unit 2 that travels from first irradiation unit 2 toward first imaging unit 4. This can prevent the light emitted from first irradiation unit 2 from directly entering first imaging unit 4. This can prevent a determination that egg E has a crack or the like from being erroneously made as the light emitted from first irradiation unit 2 directly enters first imaging unit 4.

Furthermore, second irradiation unit 3 is disposed on the end E2 side of egg E. Second imaging unit 5 disposed on a side opposite to end E2 of egg E, or the end E1 side. Egg E will be located on a line connecting second imaging unit 5 and second irradiation unit 3. With this arrangement, when egg E irradiated with light by second irradiation unit 3 is imaged by second imaging unit 5, egg E will obstruct a component of light emitted from second irradiation unit 3 that travels from second irradiation unit 3 toward second imaging unit 5. This can prevent the light emitted from second irradiation unit 3 from directly entering second imaging unit 5. This can prevent a determination that egg E has a crack or the like from being erroneously made as the light emitted from second irradiation unit 3 directly enters second imaging unit 5.

Further, egg inspection apparatus 1 as described above is provided with black reflection suppressing portion 9 on the side of the upper surface of protective member 8 disposed below conveyor unit 6. With this arrangement, reflection suppressing portion 9 can weaken a component of light emitted from first irradiation unit 2 or second irradiation unit 3, having reached conveyor unit 6, and reflected by a lower side of egg E or the like, that reaches an upper surface of protective member 8 and is reflected, and reflection suppressing portion 9 can thus suppress re-arrival of the component at conveyor unit 6. This can suppress entrance of the component that is reflected on the upper surface of protective member 8 into first imaging unit 4 or second imaging unit 5 as stray light, and thus prevent reduction in accuracy of determination. In addition, reflection suppressing portion 9 will also have a function of narrowing the light emitted from first irradiation unit 2 or second irradiation unit 3.

Further, egg inspection apparatus 1 described above is disposed in egg grading system 100 as broken egg detection unit 103 upstream of egg washing unit 104 and drying unit 105 (see Fig. 1). This allows broken or leaked eggs or the like to be screened out before they are transported to egg washing unit 104 or drying unit 105. This can prevent broken or similar eggs from contaminating egg washing unit 104 or drying unit 105, and thus also save labor for washing.

Note that the present invention is not limited to the above described embodiment.

Egg inspection apparatus 1 may be applied to inspections other than that for whether or not an egg is broken. Furthermore, the egg inspection apparatus may be disposed at an appropriate position within egg grading system 100 (see Fig. 1), as required. That is, egg inspection apparatus 1 is not only disposed upstream of egg washing unit 104 but may instead be disposed for example downstream of egg washing unit 104 and upstream of drying unit 105. Further, egg inspection apparatus 1 may be disposed downstream of drying unit 105.

Egg grading system 100 can also be modified variously as required at a portion other than egg inspection apparatus 1 serving as broken egg detection unit 103. Further, egg inspection apparatus 1 may be applied to an egg grading system without a washing unit and a drying unit.

First irradiation unit 2 and second irradiation unit 3 can be variously modified insofar as they can emit predetermined light toward eggs. For example, an irradiation unit that emits light which is transmitted through an egg is preferable, and specifically, an irradiation unit that emits light including a component of visible to infrared light is preferable. As the infrared light, for example, near infrared light having a wavelength of about 750 nm to 1500 nm is preferable, and within that wavelength range, near infrared light having a wavelength of about 780 nm to 870 nm is more preferable. Infrared light having a wavelength shorter than 780 nm is affected by eggshell color and thus less easily enters an egg, and is thus less transmitted through the egg, resulting in the egg having a poor contract against a background. On the other hand, infrared light having a wavelength longer than 870 nm would be absorbed by moisture contained in egg yolk and egg white, and accordingly, less transmitted through the egg, resulting in the egg having a poor contract against a background. Further, as the irradiation unit, an irradiation unit having a lens or the like to refract and converge light may be used. Further, an irradiation unit having a diaphragm or the like to restrict an angle of spreading of light may be used.

Further, where first irradiation unit 2 and second irradiation unit 3 are disposed is not limited as described above. For example, the irradiation unit may be disposed to be able to emit light from a position away in the direction of roller shaft 61 from one (or the other) end of an egg obliquely upward toward the opening located between rollers 62s. Specifically, the irradiation unit may be disposed at a position away in the direction of a roller shaft from a roller bearing an egg to be irradiated, and emit light toward the egg's equatorial region. In that case, it is necessary to appropriately change the position of reflection suppressing portion 9. Furthermore, the irradiation unit may be disposed under an egg other than an egg to be irradiated, and emit light toward the egg's equatorial region.

Furthermore, when the light emitted from the irradiation unit is obstructed by an egg other than an egg to be irradiated, the irradiation unit and an imaging unit that images the egg to be irradiated by the irradiation unit may be disposed on the same end side of the egg. Furthermore, when the light emitted from the irradiation unit is obstructed by a shielding object other than an egg, the irradiation unit and the imaging unit that images an egg to be irradiated by the irradiation unit may be disposed on the same end side of the egg. That is, egg inspection apparatus 1 according to the present invention may comprise a first imaging unit that images an egg from the side of the other end of the egg while the egg is irradiated with light emitted from the second irradiation unit disposed on the side of the other end of the egg, and a second imaging unit that images an egg from the side of one end of the egg while the egg is irradiated with light emitted from the first irradiation unit disposed on the side of one end of the egg.

First irradiation unit 2 and second irradiation unit 3 may be aligned in a direction substantially orthogonal to the conveying direction.

Insofar as timing of imaging by first imaging unit 4 and timing of imaging by second imaging unit 5 do not overlap, timing of emitting light by first irradiation unit 2 and timing of emitting light by second irradiation unit 3 may partially overlap. Further, all of first irradiation units 2 may be timed to concurrently emit light. Further, first irradiation unit 2 may be divided into a plurality of groups, and the groups may be timed to emit light at different times. Second irradiation unit 3 is similarly discussed.

First imaging unit 4 and second imaging unit 5 are not limited to the above example as long as they can image an egg. Further, although an image of an egg imaged by first imaging unit 4 and second imaging unit 5 is schematically shown, the image is merely an example.

Determination unit 10 may determine a condition of a surface of an egg based on a difference in brightness (or contrast) of an image of the egg. For example, if there is a portion in an image of an egg that has a significant difference in brightness (i.e., an edge), it can be determined that there is a "crack" or an "adhesion of feces" in that portion. Before edge detection is performed, preprocessing well known in the field of art may be applied to the image.

Conveyor unit 6 is not limited to a conveyor unit that transports eggs while rotating them. Further, the present invention is not limited to a conveyor unit which transports eggs placed with their longer axes extending in a direction traversing the conveying direction. Furthermore, the number of rows of conveyor unit 6 is not limited to six rows.

Reflection suppressing portion 9 is not limited to the above described manner in sofaras it comprises a material, has a shape, and the like that can weaken the light reflected by egg E or a lower surface side of conveyor unit 6. Furthermore, the location of reflection suppressing portion 9 is not limited to the upper surface of protective member 8 as long as the location is below conveyor unit 6.

It should be understood that the embodiments disclosed herein have been described for the purpose of illustration only and in a non-restrictive manner in any respect. The present invention is defined by the terms of the claims, rather than the description.

### INDUSTRIAL APPLICABILITY

The present invention is applicable to an egg inspection apparatus to determine a condition of a surface of an egg.

### REFERENCE SIGNS LIST

1: egg inspection apparatus; 2: first irradiation unit; 3: second irradiation unit; 4: first imaging unit; 5: second imaging unit; 6: conveyor unit; 8: protective member; 9: reflection suppressing portion; 10: determination unit; 11: control unit; 61: roller shaft; 62: roller; 63: opening; 100: egg grading system; 101: egg feeding unit; 102: orientation unit; 103: broken egg detection unit; 104: egg washing unit; 105: drying unit; 106: grading and collecting unit; P: image; E: egg.

## Claims

1. An egg inspection apparatus (1) comprising:
a plurality of irradiation units (2, 3) configured to emit light toward an egg;
a plurality of imaging units (4, 5) configured to image the egg irradiated with the light, including the light transmitted through the egg;
a determination unit (10) configured to determine a condition of a surface of the egg based on an image of the egg imaged by the imaging unit; and
a conveyor unit (6) configured to transport the egg while rotating the egg, wherein the irradiation units are disposed below the conveyor unit,
the plurality of irradiation units including
a first irradiation unit (2) configured to emit the light from a side of one end of the egg, and
a second irradiation unit (3) configured to emit the light from a side of the other end of the egg,
the plurality of imaging units including
a first imaging unit (4) configured to image the egg from the side of the other end of the egg, and
a second imaging unit (5) configured to image the egg from the side of the one end of the egg,
the first imaging unit imaging the egg when one of the first irradiation unit and the second irradiation unit emits the light toward the egg,
the second imaging unit imaging the egg when the other of the first irradiation unit and the second irradiation unit emits the light toward the egg, whereby
the first imaging unit (4) is configured to image the egg when the first irradiation unit (2) emits light toward the egg and the second irradiation unit (3) does not emit light toward the egg, while the second imaging unit (5) is configured to image the egg when the first irradiation unit (2) does not emit light toward the egg and the second irradiation unit (3) emits light toward the egg.

2. The egg inspection apparatus according to claim 1, further comprising:
a reflection suppressing unit disposed below the conveyor unit and configured to suppress reflection of the light.

## Patentansprüche

1. Ei-Inspektions-Vorrichtung (1), umfassend:
eine Mehrzahl von Bestrahlungseinheiten (2, 3), die so eingerichtet sind, dass sie Licht in Richtung eines Eies emittieren;
eine Mehrzahl von Bildgebungseinheiten (4, 5), die zur Abbildung des mit dem Licht bestrahlten Eies konfiguriert sind, einschließlich des durch das Ei transmittierten Lichts;
eine Bestimmungseinheit (10), die zur Bestimmung eines Zustands einer Oberfläche des Eies basierend auf einem Bild des von der Bildgebungseinheit abgebildeten Eies konfiguriert ist; und
eine Fördereinheit (6), die zum Transport des Eies während der Drehung des Eies konfiguriert ist, wobei die Bestrahlungseinheiten unterhalb der Fördereinheit angeordnet sind,
die Mehrzahl von Bestrahlungseinheiten umfassend
eine erste Bestrahlungseinheit (2), die zur Emission des Lichts von einer Seite eines Endes des Eies konfiguriert ist, und
eine zweite Bestrahlungseinheit (3), die zur Emission des Lichts von einer Seite des anderen Endes des Eies konfiguriert ist,
die Mehrzahl von Bildgebungseinheiten umfassend
eine erste Bildgebungseinheit (4), die zur Abbildung des Eies von der Seite des anderen Endes des Eies konfiguriert ist, und
eine zweite Bildgebungseinheit (5), die zur Abbildung des Eies von der Seite des einen Endes des Eies konfiguriert ist,
die erste Bildgebungseinheit bildet das Ei ab, wenn eine der ersten Bestrahlungseinheit und der zweiten Bestrahlungseinheit das Licht in Richtung des Eies emittiert,
die zweite Bildgebungseinheit bildet das Ei ab, wenn die andere der ersten Bestrahlungseinheit und der zweiten Bestrahlungseinheit das Licht in Richtung des Eies emittiert, wodurch
die erste Bildgebungseinheit (4) zur Abbildung des Eies konfiguriert ist, wenn die erste Bestrahlungseinheit (2) Licht in Richtung des Eies emittiert und die zweite Bestrahlungseinheit (3) kein Licht in Richtung des Eies emittiert, während die zweite Bildgebungseinheit (5) zur Abbildung des Eies konfiguriert ist, wenn die erste Bestrahlungseinheit (2) kein Licht in Richtung des Eies emittiert und die zweite Bestrahlungseinheit (3) Licht in Richtung des Eies emittiert.

2. Die Ei-Inspektions-Vorrichtung gemäß Anspruch 1, weiterhin umfassend:
eine Reflexionsunterdrückungseinheit, die unterhalb der Fördereinheit vorgesehen ist und zur Unterdrückung der Reflexion des Lichts konfiguriert ist.

## Revendications

1. Appareil d'inspection d'oeuf (1) comprenant :
une pluralité d'unités d'irradiation (2, 3) configurées pour émettre de la lumière vers un œuf ;
une pluralité d'unités d'imagerie (4, 5) configurées pour imager l' œuf irradié par la lumière, y compris la lumière transmise à travers l'œuf ;
une unité de détermination (10) configurée pour déterminer un état d'une surface de l'œuf sur la base d'une image de l'œuf imagée par l'unité d'imagerie ; et
une unité de transport (6) configurée pour transporter l'œuf tout en faisant tourner l'oeuf, dans laquelle les unités d'irradiation sont disposées sous l'unité de transport,
la pluralité d'unités d'irradiation comprenant
une première unité d'irradiation (2) configurée pour émettre la lumière d'un côté d'une extrémité de l'œuf, et
une seconde unité d'irradiation (3) configurée pour émettre la lumière à partir d'un côté de l'autre extrémité de l'œuf,
la pluralité d'unités d'imagerie comprenant
une première unité d'imagerie (4) configurée pour imager l'œuf du côté de l'autre extrémité de l'œuf, et
une seconde unité d'imagerie (5) configurée pour imager l'œuf du côté de la première extrémité de l'œuf,
la première unité d'imagerie image l'œuf lorsque l'une de la première unité d'irradiation et de la seconde unité d'irradiation émet la lumière vers l'œuf,
la seconde unité d'imagerie image l'œuf lorsque l'autre de la première unité d'irradiation et de la seconde unité d'irradiation émet la lumière vers l'œuf, moyennant quoi
la première unité d'imagerie (4) est configurée pour imager l'œuf lorsque la première unité d'irradiation (2) émet de la lumière vers l'œuf et que la seconde unité d'irradiation (3) n'émet pas de lumière vers l'œuf, tandis que la seconde unité d'imagerie (5) est configurée pour imager l'oeuf lorsque la première unité d'irradiation (2) n'émet pas de lumière vers l'œuf et que la seconde unité d'irradiation (3) émet de la lumière vers l'œuf,

2. Appareil d'inspection d'oeuf selon la revendication 1, comprenant en outre :
une unité de suppression de réflexion disposée sous l'unité de transport et configurée pour supprimer la réflexion de la lumière.
